# EUROPÄISCHE PATENTANMELDUNG

(11) **EP 0 718 036 A1**
(43) Veröffentlichungstag der Anmeldung: **26.06.1996**
(21) Anmeldenummer: 95117578.5
(22) Anmeldetag: 08.11.1995
(51) Int. Cl.: B01J 23/02, C07C 5/25, C07C 11/02

(54) **Katalysator und Verfahren zur Isomerisierung von aliphatischen C4-C10-Monoolefinen**

(30) Priorität: 21.12.1994 DE 4445680
(71) Anmelder: HÜLS AKTIENGESELLSCHAFT, D-45764 Marl (DE)
(72) Erfinder: Kaizik, Alfred, Dr., D-45772 Marl (DE); Droste, Wilhelm, Dr., D-45770 Marl (DE)

(57) **Zusammenfassung**

Die Erfindung betrifft einen Katalysator und ein Verfahren zur Isomerisierung von aliphatischen C₄-C₁₀-Monoolefinen mit innenständiger Doppelbindung zu den entsprechenden Monoolefinen mit endständiger Doppelbindung, wobei der Katalysator ein Erdalkalioxid auf Aluminiumoxid enthält.

## Beschreibung

Die Erfindung betrifft einen Katalysator zur Isomerisierung von aliphatischen C₄-C₁₀-Monoolefinen mit innenständiger Doppelbindung zu den entsprechenden Monoolefinen mit endständiger Doppelbindung sowie ein Verfahren zur Isomerisierung von aliphatischen C₄-C₁₀-Monoolefinen mit innenständiger Doppelbindung zu den entsprechenden Monoolefinen mit endständiger Doppelbindung.

Monoolefine mit endständiger Doppelbindung, sogenannte alpha- oder terminal-Olefine, sind wertvolle Ausgangsstoffe für eine Reihe technisch wichtiger Prozesse, wie z. B. die Hydroformylierung, Sulfonierung und Oligomerisierung. Im Vergleich zu Monoolefinen mit innenständiger Doppelbindung weisen Monoolefine mit endständiger Doppelbindung eine deutlich höhere Reaktivität auf.

Von besonderer wirtschaftlicher Bedeutung sind C₄- bis C₈-Monoolefine mit endständiger Doppelbindung, wie 1-Buten, 1-Hexen und 1-Octen, die als Comonomere mit Ethylen bei der Herstellung von linearem Low-Density-Polyethylen (LLDPE) Verwendung finden. 1-Buten wird darüber hinaus für die Herstellung von 1-Polybuten und Butenoxid eingesetzt.

Monoolefine mit endständiger Doppelbindung, wie z. B. 1-Buten, können durch katalytische Doppelbindungsisomerisierung der entsprechenden Monolefine mit innenständiger Doppelbindung hergestellt werden.

Es ist bekannt, z. B. aus der US-PS 3 642 933, US-PS 4 229 610 und DE-PS 33 19 171, Monoolefine mit innenständiger Doppelbindung zu Monoolefinen mit endständiger Doppelbindung, insbesondere 2-Buten zu 1-Buten, katalytisch in der Gasphase an unterschiedlichen Katalysatorsystemen zu isomerisieren.

So werden gemäß US-PS 3 642 933 Zirkonoxid auf Aluminiumoxid, gemäß US-PS 4 229 610 Aluminiumoxid mit Siliziumdioxid und mit einem relativ geringen Gehalt an Natriumoxid sowie gemäß DE-PS 33 19 171 Calcium- und/oder Bariumoxid auf mit Kieselsäure stabilisierter γ-Tonerde als Katalysatorsysteme eingesetzt.

DE-PS 33 19 099 lehrt einen Katalysator mit mit Kieselsäure stabilisierter γ-Tonerde als Träger, der mit einer Calcium- oder Bariumsalzlösung imprägniert wird, wobei dieser Katalysator zur Isomerisierung von 2-Buten zu 1-Buten verwendet werden kann.

Weiterhin ist aus US-PS 4 289 919 ein Verfahren zur Isomerisierung von Monolefinen, insbesondere von 2-Buten, an einem mit Manganoxid modifizierten Aluminiumoxid-Katalysator bekannt, bei dem gemäß den in der Patentschrift aufgeführten Beispielen relativ hohe Reaktionstemperaturen und eine Verdünnung mit Stickstoff bei der Isomerisierung erforderlich sind.

Schließlich wird in US-PS 3 479 415 offenbart, 2-Butene bei Temperaturen von 450 bis 500 °C an einem Natriumoxid-armen, makroporösen Siliziumoxid-Katalysator unter Verwendung von Wasserdampf als Verdünnungsmittel zu 1-Buten zu isomerisieren. Durch die Verdünnung des C₄-Kohlenwasserstoffeinsatzgemisches mit Wasserdampf konnte die Bildung von desaktivierenden Ablagerungen des Kohlenstoffs auf dem Katalysator verlangsamt und dadurch die Katalysatorstandzeit verlängert werden.

Die bekannten Verfahren zur Isomerisierung von 2-Buten zu 1-Buten haben jedoch die Nachteile, daß die Produktreinheit durch die Bildung von Nebenprodukten, wie z. B. 1,3-Butadien, Isobuten und Hochsieder, nicht ausreichend, die Ausbeute unbefriedigend oder der eingesetzte Katalysator nur schwer zu regenerieren ist. Bedingt durch diese verfahrenstechnischen Schwierigkeiten haben die Verfahren gemäß dem Stand der Technik bisher keine großtechnische Anwendung gefunden.

Der Erfindung liegt daher die Aufgabe zugrunde, einen Katalysator zur Isomerisierung von aliphatischen C₄-C₁₀-Monoolefinen mit innenständiger Doppelbindung zu den entsprechenden Monoolefinen mit endständiger Doppelbindung zu entwickeln, mit welchem sich höhere Selektivitäten und Umsätze erzielen lassen. Des weiteren soll sich der Katalysator auf einfache Weise regenerieren lassen.

Es wurde nun überraschenderweise gefunden, daß mit einem Katalysator, der ein Erdalkalioxid auf Aluminiumoxid enthält, besonders hohe Selektivitäten und Umsätze bei der Isomerisierung von aliphatischen C₄-C₁₀-Monoolefinen mit innenständiger Doppelbindung zu den entsprechenden Monoolefinen mit endständiger Doppelbindung erhalten werden.

Außerdem läßt sich ein erfindungsgemäßer, desaktivierter Katalysator besonders einfach regenerieren, indem man die sich gebildeten Kohlenstoffablagerungen beispielsweise durch Abbrennen entfernt. Hierbei kann praktisch die ursprüngliche Aktivität des erfindungsgemäßen Katalysators wi dererlangt werden.

Gegenstand der vorliegenden Erfindung ist daher ein Katalysator zur Isomerisierung von aliphatischen C₄-C₁₀-Monoolefinen mit innenständiger Doppelbindung zu den entsprechenden Monoolefinen mit endständiger Doppelbindung, der dadurch gekennzeichnet ist, daß der Katalysator ein Erdalkalioxid auf Aluminiumoxid enthält.

Des weiteren ist Gegenstand der vorliegenden Erfindung ein Verfahren zur Isomerisierung von aliphatischen C₄-C₁₀-Monoolefinen mit innenständiger Doppelbindung zu den entsprechenden Monoolefinen mit endständiger Doppelbindung, das dadurch gekennzeichnet ist, daß die Isomerisierung an einem Katalysator nach den Ansprüchen 1 bis 6 durchgeführt wird.

Vorzugsweise enthält der erfindungsgemäße Katalysator ein Erdalkalioxid auf saurem Aluminiumoxid.

Der Gehalt des erfindungsgemäßen Katalysators an Erdalkalioxid beträgt vorzugsweise 0,5 bis 20 Gew.-%, bezogen auf den gesamten Katalysator, besonders vorzugsweise 4 bis 12 Gew.-%, bezogen auf den gesamten Katalysator.

Bevorzugt enthält der erfindungsgemäße Katalysator Magnesiumoxid und/oder Calciumoxid und/oder Strontiumoxid und/oder Bariumoxid auf Aluminiumoxid, besonders bevorzugt Strontiumoxid auf Aluminiumoxid.

Es ist also eine besonders bevorzugte Variante des erfindungsgemäßen Katalysators, daß als Erdalkalioxid Strontiumoxid für die Modifizierung des Aluminiumoxids verwendet wird.

Der Hauptbestandteil des erfindungsgemäßen Katalysators ist geeigneterweise ein aktiviertes, oberflächenreiches Aluminiumoxid, das sowohl in der γ- als auch in der η-Kristallform vorliegen kann. γ-Aluminiumoxid wird aufgrund seiner erwünschten spezifischen Oberfläche, die im allgemeinen im Bereich von 80 bis 350 m²/g liegt, vorzugsweise verwendet. Besonders geeignet sind Aluminiumoxide mit einer Oberfläche von etwa 120 m²/g bis zu etwa 300 m²/g.

Das für den erfindungsgemäßen Katalysator eingesetzte Aluminiumoxid kann sowohl praktisch natriumoxidfrei sein, wie auch geringere Mengen an Natriumoxid bis zu ca. 1,0 Gew.-% Na₂O enthalten.

Das für die Herstellung des erfindungsgemäßen Katalysators verwendete Aluminiumoxid kann beispielsweise in Form von Strangextrudaten oder Kugeln vorliegen. Es können auch andere Formen in Abhängigkeit von dem anzuwendenden Verfahren verwendet werden.

Für die Herstellung des erfindungsgemäßen Katalysators sind die verschiedensten bekannten Verfahren geeignet.

Beispielsweise kann die Herstellung des erfindungsgemäßen Katalysators durch Tränken oder Imprägnieren des Aluminiumoxids mit wäßrigen Lösungen der entsprechenden Salze der Erdalkalimetalle erfolgen. Vorzugsweise werden hierzu wäßrige Lösungen von Erdalkalimetallnitraten oder -acetaten verwendet. Das mit Erdalkalimetall-Salz imprägnierte oder getränkte Aluminiumoxid kann bei Temperaturen von 80 bis 100 °C getrocknet und anschließend bei Temperaturen von 400 bis 550 °C kalziniert werden. Vorzugsweise wird der erfindungsgemäße Katalysator zur Isomerisierung von 2-Buten zu 1-Buten eingesetzt.

Die Isomerisierung von aliphatischen C₄-C₁₀-Monoolefinen mit innenständiger Doppelbindung zu den entsprechenden Monoolefinen mit endständiger Doppelbindung wird vorzugsweise bei Temperaturen von 150 bis 550 °C, besonders vorzugsweise bei Temperaturen von 350 bis 450 °C, durchgeführt.

Temperaturen von über 550 °C führen bei der Isomerisierung von 2-Buten aufgrund der Bildung von Nebenprodukten zur Verschlechterung der 1-Buten-Selektivität.

Die Isomerisierung kann sowohl in der Flüssigphase als auch in der Gasphase erfolgen.

Vorzugsweise wird die Isomerisierung in der Gasphase durchgeführt.

Mit Hilfe des erfindungsgemäßen Verfahrens läßt sich in besonders geeigneter Weise 2-Buten zu 1-Buten isomerisieren.

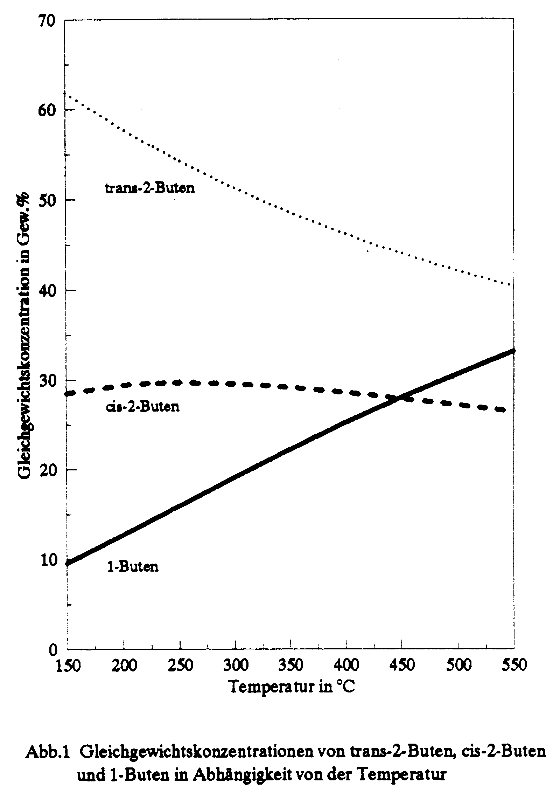

Die Isomerisierung von 2-Buten zu 1-Buten ist durch das thermodynamische Gleichgewicht der n-Buten-Isomeren begrenzt. Die auf der Basis der thermodynamischen Daten (D. Stull, "The Chemical Thermodynamics of Organic Compounds", J. Wiley, New York 1969) berechneten Gleichgewichtskonzentrationen der n-Buten-Isomeren 1-Buten (durchgezogene Linie), cis-2-Buten (gestrichelte Linie) und trans-2-Buten (gepunktete Linie) sind als Funktionen der Temperatur in der Abb. 1 dargestellt. Die Kurve für die Gleichgewichtskonzentrationen von 1-Buten entspricht den 2-Buten-Gleichgewichtsumsätzen. Das Gleichgewicht der 2-Buten-Isomerisierung wird durch hohe Temperaturen begünstigt. Die maximal erzielbaren Ausbeuten an 1-Buten (2-Buten-Umsatz x Selektivität) sind bei einem einzigen Reaktordurchgang durch das thermodynamische Gleichgewicht bei einer Temperatur von 400 °C auf etwa 22 % und bei einer Temperatur von 500 °C auf etwa 30 % begrenzt.

Die mit der vorliegenden Erfindung erzielbaren Vorteile bestehen insbesondere darin, daß z. B. mit Hilfe des erfindungsgemäßen Katalysators die thermodynamisch maximal möglichen 2-Buten-Umsätze bei einer praktisch 100 %igen 1-Buten-Selektivität im breiten Temperaturbereich erhalten werden können.

Das im erfindungsgemäßen Verfahren erhaltene 1-Buten ist praktisch frei von iso-Buten und 1,3-Butadien, so daß keine aufwendige nachgeschaltete Reinigungsstufe zur Abtrennung dieser unerwünschten Nebenprodukte erforderlich ist.

Im erfindungsgemäßen Verfahren können sowohl reines 2-Buten als auch 2-Buten-haltige Kohlenwasserstoffgemische als Einsatzstoffe verwendet werden.

So können im erfindungsgemäßen Verfahren geeigneterweise 2-Buten-haltige C₄-Kohlenwasserstoffgemische (C₄-Schnitte) eingesetzt werden, die im allgemeinen einen Gehalt an 2-Butenen von 40 bis 100 Gew.-%, insbesondere von 50 bis 65 Gew.-%, aufweisen. 2-Buten kann als cis-2-Buten oder trans-2-Buten im C₄-Schnitt vorliegen. In der Regel enthalten die verfügbaren, einzusetzenden C₄-Schnitte ein Gemisch aus cis-2-Buten und trans-2-Buten. Neben den 2-Butenen können die C₄-Schnitte auch n-Butan und geringe Mengen an 1-Buten aufweisen. Die Wirtschaftlichkeit der erfindungsgemäßen 2-Buten-Isomerisierung wird maßgebend durch den Gehalt an 2-Butenen in dem eingesetzten C₄-Schnitt bestimmt. Je höher der Gehalt an 2-Buten im C₄-Schnitt ist, desto höher sind die erzielbaren Raum-Zeit-Ausbeuten. Im allgemeinen wird kein reines 2-Buten, sondern ein 2-Buten-haltiges C₄-Kohlenwasserstoffgemisch für die erfindungsgemäße Isomerisierung von 2-Buten zu 1-Buten eingesetzt. Insbesondere wird der von Butadien, Isobuten und 1-Buten befreite Crack-C₄-Schnitt - das sogenannte Raffinat III - für die erfindungsgemäße Isomerisierung von 2-Buten zu 1-Buten verwendet.

Für das erfindungsgemäße Verfahren wird vorzugsweise ein Festbettreaktor eingesetzt. Es können auch andere Reaktortypen, wie z. B. ein Wirbelbettreaktor oder ein Wanderbettreaktor, verwendet werden.

Das erfindungsgemäße Verfahren läßt sich bei Atmosphärendruck durchführen. Es können jedoch auch höhere Reaktionsdrücke angewandt werden. Die Druckfahrweise im erfindungsgemäßen Verfahren ist insbesondere dann zweckmäßig, wenn beispielsweise das Produkt aus dem erfindungsgemäßen Verfahren noch einer Trennstufe zugeführt wird, die ebenfalls unter Druck betrieben wird. So kann man z. B. einen C₄-Schnitt nach erfolgter erfindungsgemäßer Isomerisierung von 2-Buten zu 1-Buten noch einer Trennstufe zuführen, in der 1-Buten und 2-Buten voneinander bei z. B. einer Temperatur von 60 °C und einem Druck von 7 bar abs. destillativ getrennt werden.

Die erfindungsgemäße Isomerisierung von 2-Buten zu 1-Buten erfolgt vorzugsweise bei Temperaturen von 300 bis 550 °C und bei Gas-Raumgeschwindigkeiten (gas hourly space velocity = GHSV) von 2 000 bis 8 000 h⁻¹, besonders vorzugsweise bei Temperaturen von 350 bis 500 °C und bei GHSV-Werten von 2 500 bis 4 000 h⁻¹.

Bei Nachlassen der Aktivität und Selektivität des erfindungsgemäßen Katalysators infolge von Kohlenstoffablagerungen auf dem Katalysator wird dieser zweckmäßigerweise regeneriert. Ein vorteilhaftes Verfahren zur Katalysatorregenerierung besteht darin, daß man die Kohlenstoffablagerungen auf dem desaktivierten Katalysator in Sauerstoff enthaltenden Gasen, vorzugsweise in Luft, abbrennt. Es kann zweckmäßig sein, hierbei die Luft mit Stickstoff zu verdünnen. Die Katalysatorregenerierung wird im allgemeinen bei Temperaturen von 350 bis 600 °C, vorzugsweise von 400 bis 450 °C, durchgeführt. Hierdurch lassen sich in der Regel die Anfangsaktivität und die Anfangsselektivität des erfindungsgemäßen Katalysators in einfacher Weise zurückgewinnen.

Die Erfindung wird durch die folgenden Beispiele näher erläutert.

Die erfindungsgemäße Isomerisierung von 2-Buten zu 1-Buten an den erfindungsgemäßen Katalysatoren wurde unter isothermen Bedingungen in einem Rohrreaktor mit 3,0 cm Innendurchmesser und einer Länge von 80 cm in der Gasphase durchgeführt. Als Edukt wurde ein 2-Buten-haltiger C₄-Schnitt (Raffinat III) mit ca. 62 Gew.-% 2-Buten, ca. 37 Gew.-% n-Butan und ca. 1 Gew.-% 1-Buten eingesetzt.

Die quantitative und qualitative Bestimmung der Einzelkomponenten im Edukt- und Produktgemisch erfolgte gaschromatographisch.

### Beispiel 1

Der erfindungsgemäße Katalysator A wurde wie folgt hergestellt: 100 g γ-Aluminiumoxid mit einer spezifischen Oberfläche von 250 m²/g und einem Na₂O-Gehalt von < 300 Gew.-ppm in Form von 1,1 mm x 3,0 mm Strangextrudaten wurden mit 130 ml einer wäßrigen Lösung, die 13 g Strontiumnitrat enthielt, imprägniert. Die imprägnierten Al₂O₃-Strangextrudate wurden bei einer Temperatur von 80 °C 16 h lang in einem Trockenschrank getrocknet und anschließend 5 h lang bei einer Temperatur von 450 °C in einem Umluft-Muffelofen kalziniert. Man erhielt den Katalysator A mit einem Gehalt von 6 Gew.-% SrO auf γ-Al₂O₃. Der auf diese Weise hergestellte Katalysator A wurde für die Isomerisierung von 2-Buten zu 1-Buten eingesetzt, wobei die Aktivitätstestung in dem oben beschriebenen Rohrreaktor mit Raffinat III als Edukt durchgeführt wurde.

Die 2-Buten-Umsätze und die 1-Buten-Selektivitäten sowie die Bildung der Nebenprodukte wurden bei einer Gas-Raumgeschwindigkeit von 2 800 h⁻¹ und bei Temperaturen von 450 °C und 500 °C bestimmt. Das als Edukt verwendete Raffinat III hatte die folgende Zusammensetzung:

| | |
|---|---|
| n-Butan | 37,25 Gew.-% |
| Cyclobutan | 0,188 Gew.-% |
| trans-2-Buten | 37,34 Gew.-% |
| cis-2-Buten | 24,54 Gew.-% |
| 1-Buten | 0,484 Gew.-% |
| i/n-Pentan | 0,192 Gew.-% |

Die Ergebnisse der Aktivitätstestungen, wie Zusammensetzung der Produkte nach Isomerisierung, 2-Buten-Umsätze und 1-Buten-Selektivitäten, sind für die beiden Temperaturen von 450 °C und 500 °C in der nachfolgenden Tabelle 1 aufgeführt:

**Tabelle 1**

| Ergebnisse der Aktivitätstestungen des Katalysators A bei der Isomerisierung von 2-Buten zu 1-Buten in Raffinat III | | |
|---|---|---|
| Einzelkomponenten | Zusammensetzung der Produkte nach Isomerisierung in [Gew.-%] bei | |
| | 450 °C | 500 °C |
| Methan | 0,002 | 0,021 |
| Ethan/Ethen | - | 0,025 |
| Propan | - | - |
| Propen | 0,004 | 0,055 |
| n-Butan | 37,20 | 37,16 |
| Cyclobutan | 0,141 | 0,059 |
| trans-2-Buten | 25,46 | 24,17 |
| cis-2-Buten | 20,07 | 19,82 |
| 1-Buten | 16,84 | 18,35 |
| i/n-Pentan | 0,280 | 0,275 |
| Hochsieder (C₅⁺) | - | - |
| i-Buten | - | - |
| 1,3-Butadien | - | 0,055 |
| 2-Buten-Umsatz in [%] | 26,4 | 28,9 |
| 1-Buten-Selektivität | | |
| in [%] | 100,0 | 99,8 |

Die in der Tabelle 1 aufgeführten Ergebnisse zeigen, daß durch eine gezielte Modifizierung von γ-Aluminiumoxid mit Strontiumoxid ein ausgezeichneter Katalysator für die Isomerisierung von 2-Buten zu 1-Buten hergestellt werden kann.

So werden mit Hilfe des Katalysators A praktisch 100 %ige 1-Buten-Selektivitäten erzielt. Es wird kein iso-Buten durch eine Skelett-Isomerisierung der n-Butene gebildet. Auch die Bildung von weiteren störenden Nebenprodukten, wie z. B. 1,3-Butadien, Leicht- und Hochsiedern, wird fast völlig zurückgedrängt. Die erzielten 2-Buten-Umsätze kommen den theoretischen 2-Buten-Gleichgewichtsumsätzen äußerst nahe.

Der Katalysator A wurde einem Alterungstest bei einer Temperatur von 550 °C unterworfen. Hierbei wurden 35 Reaktionszyklen von insgesamt 280 h und 35 Regenerationszyklen von insgesamt 140 h durchgeführt, ohne daß ein Aktivitätsverlust aufgetreten ist. Der Katalysator A wurde dabei jeweils mit Luft bei einer Temperatur von 450 °C regeneriert.

### Beispiel 2

Der erfindungsgemäße Katalysator B wurde wie folgt hergestellt:
Ein natriumreicheres η-Aluminiumoxid mit einer spezifischen Oberfläche von 150 m²/g und einem Gehalt an Na₂O von 0,78 Gew.-% wurde in Form von 1,5 mm x 3,5 mm Strangextrudaten wie in Beispiel 1 mit einer wäßrigen Strontiumnitrat-Lösung imprägniert und anschließend thermisch behandelt. Der so hergestellte Katalysator B enthielt 4 Gew.-% Strontiumoxid auf η-Al₂O₃. Der Katalysator B wurde für die Isomerisierung von 2-Buten zu 1-Buten eingesetzt und bei einer Gas-Raumgeschwindigkeit von 2 390 h⁻¹ sowie bei Temperaturen von 450 °C und 495 °C auf seine Aktivität hin getestet. Als Edukt wurde ein Raffinat III mit folgender Zusammensetzung verwendet:

| | |
|---|---|
| n-Butan | 35,88 Gew.-% |
| Cyclobutan | 0,206 Gew.-% |
| trans-2-Buten | 37,33 Gew.-% |
| cis-2-Buten | 25,05 Gew.-% |
| 1-Buten | 1,070 Gew.-% |
| i/n-Pentan | 0,413 Gew.-% |
| Hochsieder (C₅⁺) | 0,041 Gew.-% |

Die Ergebnisse der Aktivitätstestungen, wie Zusammensetzung der Produkte nach Isomerisierung, 2-Buten-Umsätze und 1-Buten-Selektivitäten, sind für die beiden Temperaturen von 450 °C und 500 °C in der nachfolgenden Tabelle 2 aufgeführt:

**Tabelle 2**

| Ergebnisse der Aktivitätstestungen des Katalysators B bei der Isomerisierung von 2-Buten zu 1-Buten im Raffinat III | | |
|---|---|---|
| Einzelkomponenten | Zusammensetzung der Produkte nach Isomerisierung in [Gew.-%] bei | |
| | 450 °C | 495 °C |
| Methan | 0,002 | 0,019 |
| Ethan | - | 0,008 |
| Ethen | - | 0,011 |
| Propen | 0,003 | 0,042 |
| n-Butan | 35,81 | 35,75 |
| Cyclobutan | 0,177 | 0,167 |
| trans-2-Buten | 27,13 | 25,94 |
| cis-2-Buten | 19,63 | 19,41 |
| 1-Buten | 16,79 | 18,10 |
| i/n-Pentan | 0,412 | 0,511 |
| Hochsieder (C₅⁺) | 0,041 | 0,040 |
| i-Buten | - | - |
| 1,3-Butadien | - | 0,055 |
| 2-Buten-Umsatz in [%] | 24,8 | 27,3 |
| 1-Buten-Selektivität in [%] | 100,0 | 100,0 |

Aus den in Tabelle 2 zusammengestellten Ergebnissen ist zu ersehen, daß eine Belegung von Na₂O-reicherem η-Aluminiumoxid mit Strontiumoxid ebenfalls zu einem sehr aktiven und selektiven Isomerisierungskatalysator, nämlich dem Katalysator B, führt. Die 1-Buten-Selektivitäten erreichten praktisch wieder 100 %. Durch eine Modifizierung des η-Al₂O₃ mit SrO konnte insbesondere die Grundaktivität des ηAl₂O₃ bei niedrigen Temperaturen deutlich verbessert werden.

Bei einer Reaktionstemperatur von ca. 450 °C werden an dem Katalysator B kein i-Buten und kein 1,3-Butadien gebildet, bei einer Temperatur von 495 °C entstehen nur Spuren an 1,3-Butadien. Der Katalysator B läßt sich wie der Katalysator A auf einfache Weise mit Luft bzw. einem Luft/Stickstoff-Gemisch bei einer Temperatur von 450 °C regenerieren, wobei er praktisch seine ursprüngliche Aktivität wiedererlangt.

## Patentansprüche

1. Katalysator zur Isomerisierung von aliphatischen C₄-C₁₀-Monoolefinen mit innenständiger Doppelbindung zu den entsprechenden Monoolefinen mit endständiger Doppelbindung,
dadurch gekennzeichnet,
daß der Katalysator ein Erdalkalioxid auf Aluminiumoxid enthält.

2. Katalysator nach Anspruch 1,
dadurch gekennzeichnet,
daß der Katalysator ein Erdalkalioxid auf saurem Aluminiumoxid enthält.

3. Katalysator nach den Ansprüchen 1 und 2,
dadurch gekennzeichnet,
daß der Gehalt an Erdalkalioxid 0,5 bis 20 Gew.-%, bezogen auf den gesamten Katalysator, beträgt.

4. Katalysator nach Anspruch 3,
dadurch gekennzeichnet,
daß der Gehalt an Erdalkalioxid 4 bis 12 Gew.-%, bezogen auf den gesamten Katalysator, beträgt.

5. Katalysator nach den Ansprüchen 1 bis 4,
dadurch gekennzeichnet,
daß der Katalysator Magnesiumoxid und/oder Calciumoxid und/oder Strontiumoxid und/oder Bariumoxid auf Aluminiumoxid enthält.

6. Katalysator nach Anspruch 5,
dadurch gekennzeichnet,
daß der Katalysator Strontiumoxid auf Aluminiumoxid enthält.

7. Katalysator nach den Ansprüchen 1 bis 6,
dadurch gekennzeichnet,
daß der Katalysator zur Isomerisierung von 2-Buten zu 1-Buten eingesetzt wird.

8. Verfahren zur Isomerisierung von aliphatischen C₄-C₁₀-Monoolefinen mit innenständiger Doppelbindung zu den entsprechenden Monoolefinen mit endständiger Doppelbindung,
dadurch gekennzeichnet,
daß die Isomerisierung an einem Katalysator nach den Ansprüchen 1 bis 6 durchgeführt wird.

9. Verfahren nach Anspruch 8,
dadurch gekennzeichnet,
daß die Isomerisierung bei Temperaturen von 150 bis 550 °C durchgeführt wird.

10. Verfahren nach Anspruch 9,
dadurch gekennzeichnet,
daß die Isomerisierung bei Temperaturen von 350 bis 450 °C durchgeführt wird.

11. Verfahren nach den Ansprüchen 8 bis 10,
dadurch gekennzeichnet,
daß die Isomerisierung in der Gasphase durchgeführt wird.

12. Verfahren nach den Ansprüchen 8 bis 11,
dadurch gekennzeichnet,
daß 2-Buten zu 1-Buten isomerisiert wird.
